# EUROPÄISCHE PATENTSCHRIFT

(11) **EP 1 476 140 B1**
(45) Veröffentlichungstag und Bekanntmachung des Hinweises auf die Patenterteilung: **04.04.2018**
(21) Anmeldenummer: 03742449.6
(22) Anmeldetag: 04.02.2003
(51) Int. Cl.: A61K 47/02, A61K 9/70, A61K 9/46, A61K 9/20, A61K 9/00, A61K 31/451

(54) **GESCHMACKSMASKIERTE FILM- ODER OBLATENFÖRMIGE ARZNEIZUBEREITUNG**
TASTE-MASKED FILM-TYPE OR WAFER-TYPE MEDICINAL PREPARATION
PRÉPARATION PHARMACEUTIQUE A GOÛT MASQUÉ SE PRÉSENTANT SOUS LA FORME DE FILMS OU DE PLAQUES

(30) Priorität: 21.02.2002 DE 10207394
(43) Veröffentlichungstag der Anmeldung: 17.11.2004
(73) Patentinhaber: LTS LOHMANN Therapie-Systeme AG, 56626 Andernach (DE)
(72) Erfinder: RADEMACHER, Tina, 53498 Bad Breisig (DE); SEIBERTZ, Frank, 56598 Rheinbrohl (DE); BRANDT, Petra, 25421 Pinneberg (DE); VON FALKENHAUSEN, Christian, 53340 Meckenheim (DE); KRUMME, Markus, Randolph, NJ 07869 (US)
(86) Internationale Anmeldenummer: PCT/EP2003/001052
(87) Internationale Veröffentlichungsnummer: WO 2003/070227

(56) Entgegenhaltungen:
- EP-A1- 0 418 043
- EP-A1- 0 460 588
- WO-A-99/37308
- WO-A1-01/70194
- WO-A1-97/44023
- WO-A2-01/78692
- WO-A2-02/43657
- WO-A2-93/20138
- GB-A- 1 226 821
- GB-A- 1 510 999
- US-A1- 2001 006 677
- "Bupivacaine", , 2 November 2010 (2010-11-02), XP55012237, Retrieved from the Internet: URL:http://www.ycgmp.com/en/product/pharma ceutical_raw_materials_and_interm/2010/110 2/57718.html [retrieved on 2011-11-16]
- HUNG T ET AL: "Topical bupivacaine in paediatric day-case tonsillectomy: A prospective randomized controlled trial", JOURNAL OF LARYNGOLOGY AND OTO, CAMBRIDGE UNIVERSITY PRESS, GB, vol. 116, no. 1, 1 January 2002 (2002-01-01), pages 33-36, XP008145307, ISSN: 0022-2151, DOI: 10.1258/0022215021910311

## Beschreibung

Die Erfindung betrifft dünne, film- oder oblatenförmige, oral zu verabreichende Wirkstoffzubereitungen zur Verabreichung von Wirkstoffen, vorzugsweise von Arzneimittelwirkstoffen. Die Wirkstoffe werden dabei bevorzugt über die Mundschleimhaut verabreicht. Werden die Wirkstoffzubereitungen geschluckt, so werden die Wirkstoffe im Magen und/oder im Darm freigesetzt.

Bei herkömmlichen Darreichungsformen, wie z. B. Tabletten, welche im Magen zerfallen und dort den Wirkstoff freisetzen, tritt die Wirkung des Arzneimittels in der Regel erst mit erheblicher zeitlicher Verzögerung ein. Bei Tabletten, die bereits im Mund zerfallen und deren Wirkstoff über die Mundschleimhaut aufgenommen wird, ist dieser Nachteil zwar abgemildert, allerdings ist dabei zu beachten, daß ein beträchtlicher Anteil der Wirkstoffzubereitung mit dem Speichel in den Magen gelangt und deshalb nicht für eine schnelle Resorption via Mundschleimhaut zur Verfügung steht. Zudem kommt es nach gastrointestinaler Resorption des Wirkstoffes zu einem relativ raschen metabolischen Abbau des Wirkstoffs in der Leber ("First-pass"-Effekt).

Aus diesen und anderen Gründen sind dünne Darreichungsformen, wie z. B. film- oder oblatenförmige Zubereitungen, von Vorteil. Durch die geringe Dicke im Vergleich zur Fläche ergibt sich ein kurzer Diffusionsweg, wenn eine solche Arzneiform beispielsweise auf die Mundschleimhaut appliziert wird. Dies führt zu einer raschen Freisetzung des Wirkstoffs, welcher schnell und direkt über die Mundschleimhaut resorbiert werden kann. Flache Wirkstoffträger wurden bereits für verschiedene Zwecke entwickelt und hergestellt. Als grundlegend für diese Darreichungsform kann die DE-OS 27 46 414 angesehen werden, die ein folienartiges Band aus Wirkstoff, Bindemittel und weiteren Hilfsstoffen beschreibt. Dabei besteht aufgrund der homogenen Dicke, Dichte und Breite ein direkter Zusammenhang zwischen einer Längeneinheit des Bandes und der darin enthaltenen Wirkstoffdosis. Die Vorteile der kontinuierlichen Dosierbarkeit wurden auch von anderen Anmeldern erkannt und in speziellen Einzelvarianten beschrieben. So beschreibt DE-PS 36 30 603 ein flächiges Trägermaterial, z. B. in Form eines Trennpapiers mit einer wirkstoffhaltigen Beschichtung, wobei letztere nach Vorzerteilung in Dosiereinheiten vom Trägermaterial dosisweise abziehbar ist.

In der DE-OS 196 52 188 wird eine flache Arzneizubereitung beschrieben, die für die Applikation und Freisetzung des Opiat-Analgetikums Buprenorphin in der Mundhöhle geeignet ist. Allerdings wird bei dieser Darreichungsform ein Großteil der darin enthaltenen Wirkstoffmenge über den Speichel in den Magen transportiert und metabolisiert, da diese Darreichungsform nicht bzw. in nicht ausreichendem Maße mucoadhäsiv ist.

In US 2001/0006677 A1 wird ein sprudelnder, wasserlöslicher, schmelzextrudierter Dünnfilm zur gesteuerten Wirkstoffabgabe beschrieben. Ein solcher Film umfaßt ein wasserlösliches und quellfähiges Bindemittel, einen Wirkstoff, ein "effervescent couple" und optional weitere Bestandteile. Der sprudelnde Effekt wird durch das "effervescent couple", ein Brausemittel bestehend aus einer sauren und einer basischen Komponente, hervorgerufen.

Zwar sind die generellen Vorteile flacher Darreichungsformen im Stand der Technik bekannt, z. B. die bereits erwähnte schnellere Wirkstoffabgabe und einfachere Dosierbarkeit, ferner die Möglichkeit einer diskreten Einnahme, d. h. ohne Zuhilfenahme von Flüssigkeit, ferner Vorteile bei der Herstellung sowie die Möglichkeit der Bedruckung während der Herstellung, wodurch die Einnahmesicherheit erhöht werden kann.

Trotz der geschilderten Vorteile haben sich solche flächenförmige Darreichungsformen bisher kaum durchgesetzt. Vermutlich schätzen viele Hersteller von Pharmazeutika den Nutzen gegenüber herkömmlichen Darreichungsformen für zu gering ein, so daß es nicht lohnenswert erscheint, Produkte dieser Art zu entwickeln und deren arzneimittelrechtliche Zulassung zu betreiben. Insbesondere dann, wenn es sich um einen ohnehin oral applizierbaren Wirkstoff handelt, wird der Aufwand zur Entwicklung einer alternativen Darreichungsform gescheut, selbst wenn die damit verbundenen Vorteile bekannt sind.

Ein weiterer Grund dafür, daß sich flächenförmige orale Darreichungsformen bisher kaum durchgesetzt haben, besteht vermutlich auch in einer ungenügenden Patientencompliance. Vielen Wirkstoffen ist ein bitterer Geschmack zu eigen, so daß deren orale Verabreichung, insbesondere bei Resorption des Wirkstoffes über die Mundschleimhaut, mit einem unangenehmen Geschmackserlebnis einhergeht. Diese unangenehme Geschmackssensation führt zu einer geringen Akzeptanz flächenförmiger oraler Darreichungsformen bei den Patienten.

Bei Tabletten und Kapseln, die im Magen zerfallen und dort den Wirkstoff freisetzen, werden die mit dem bitteren Geschmack der Wirkstoffe einhergehenden Probleme in der Regel durch einen Überziehen der Arzneiform mit einer geschmacksneutralen Beschichtung gelöst.

Für dünne, flächenförmige Arzneizubereitungen, die den Wirkstoff in der Mundhöhle freisetzen, kommt eine Beschichtung dieser Darreichungsform mit einem geschmacksneutralen Überzug jedoch nicht in Betracht. Dieser Ansatz einer Geschmacksmaskierung fällt insbesondere bei mucoadhäsiven und/oder schnell zerfallenden flächenförmigen Darreichungsformen aus, bei denen der Wirkstoff möglichst rasch freigesetzt und von der Mundschleimhaut resorbiert werden soll oder bei Darreichungsformen, die als rein mukoadhäsives System über längere Zeiträume appliziert werden.

WO 01/70194 A1 offenbart schnell lösliche, verzehrbare Filme, die ein Ionenaustauscher-Harz als geschmacksmaskierendes Mittel sowie einen pharmazeutischen Wirkstoff, dessen Geschmack maskiert wird, enthalten, beispielsweise Dextromethorphan. Der pharmazeutische Wirkstoff wird an das Ionenaustauscherharz adsorbiert.

Die Aufgabe der Erfindung bestand deshalb darin, dünne flächenförmige Arzneizubereitungen zur Verabreichung von Wirkstoffen über die Mundschleimhaut bereitzustellen, die die Nachteile einer problematischen Geschmackssensation nicht mehr oder nur noch stark vermindert zeigen, und bei denen eine Adsorption der Wirkstoffe an Ionenaustauscherharze nicht erforderlich ist.

Erfindungsgemäß wird diese Aufgabe durch film- oder oblatenförmige Arzneizubereitungen nach Anspruch 1 sowie durch die Verwendung einer Kohlendioxid-bildenden Substanz als geschmacksmaskierender Zusatz gemäß Anspruch 22, sowie durch die in den Unteransprüchen beschriebenen bevorzugten Ausführungsformen gelöst.

Die erfindungsgemäßen Arzneizubereitungen zeichnen sich dadurch aus, daß sie eine Matrix aufweisen, die aus mindestens einem matrixbildenden Polymer gebildet ist und in welcher mindestens ein bitter schmeckender, gemäß Anspruch 1 ausgewählter Wirkstoff, der über die Mundschleimhaut oder im Magen und/oder Darm resorbiert wird, sowie mindestens eine Kohlendioxid bildende Substanz gelöst oder dispergiert ist. Die genannte Kohlendioxid bildende Substanz ist aus der Gruppe ausgewählt, die aus Natriumhydrogencarbonat, Natriumcarbonat, Kaliumcarbonat und Kaliumhydrogencarbonat besteht, und diese Substanz liegt nicht in Kombination mit einer Säurekomponente vor.

Die Verwendung von Kohlendioxidbildnern ist bereits bei medizinischen Kaugummis beschrieben worden. So beschreibt das US Patent 4,639,368, daß Kohlendioxidbildner vorzugsweise als feine Granula mit einer Größe von weniger als 10 µm in dem Grundmaterial des Kaugummies enthalten sein können.

Als Kohlendioxid-bildende Substanzen kommen für die hier offenbarten Arzneizubereitungen pharmazeutisch anwendbare ein- und zweibasige Salze der Kohlensäure, z.B. Alkalimetallhydrogen- oder Alkalimetallcarbonate, Erdalkalimetallcarbonate oder Ammoniumcarbonat und deren Mischungen in Betracht, jedoch können auch andere, physiologisch unbedenkliche Kohlendioxidbildner verwendet werden. Als erfindungsgemäße Kohlendioxid-freisetzende Substanzen sind Natriumhydrogencarbonat, Natriumcarbonat, Kaliumhydrogencarbonat oder Kaliumcarbonat zu nennen. Kohlendioxid-bildende Substanzen sind dem Fachmann bekannt und können in Kombination wirksam sein.

Bei oraler Applikation einer erfindungsgemäßen Arzneizubereitung, welche einen Kohlendioxidbildner ohne Säurezusatz enthält, werden überraschenderweise die Geschmacksempfindungen derart verändert, daß bitter schmeckende Substanzen bzw. Wirkstoffe diese unangenehme Geschmackssensation nicht mehr oder nur noch stark vermindert zeigen.

Die erfindungsgemäßen Arzneizubereitungen sind für eine Vielzahl unterschiedlicher Wirkstoffe geeignet. Voraussetzung für eine transmucosale, z. B. buccale oder sublinguale, Applikation im Mundraum ist jedoch, daß die orale Mucosa für den Wirkstoff eine ausreichende Permeabilität aufweist, unter Berücksichtigung der notwendigen Dosis. Die Permeabilität wiederum hängt in hohem Maße von den physikochemischen Eigenschaften des Wirkstoffs ab. Bei erfindungsgemäßen Arzneimittelzubereitungen, die geschluckt werden sollen, ist Voraussetzung, daß die Wirkstoffe im Magen und/oder Darm resorbiert werden. Beispiele für Wirkstoffe, die geeignet sind, um mit der hier offenbarten Arzneizubereitung verabreicht zu werden, sind Antipyretika und Analgetika, beispielsweise Ibuprofen, Acetaminophen oder Aspirin; Laxantia, beispielsweise Phenolphthaleindioctylnatriumsulfosuccinat; Appetitzügler, beispielsweise Amphetamine, Phenylpropanolamin, Phenylpropanolaminhydrochlorid oder Coffein; Antiazida, beispielsweise Calciumcarbonat; Antiasthmatika, beispielsweise Theophyllin; Antidiuretika, beispielsweise Diphenoxylathadrochlorid; Mittel gegen Blähungen, beispielsweise Simethecon; Migränemittel, beispielsweise Ergotamintartrat; Psychopharmaka, beispielsweise Haloperidol; Spasmolytika oder Sedativa, beispielsweise Phenobarbitol (mit oder ohne Atropin); Antihyperkinetika, beispielsweise Methyldopa oder Methylphenidat; Tranquilizer, beispielsweise Benzodiazepine, Hydroxinmeprobramate oder Phenothiazine; Antihistaminika, beispielsweise Astemizol, Chlorpheniraminmaleat, Pyridaminmaleat, Doxlaminsuccinat, Brompheniraminmaleat, Phenyltoloxamincitrat, Chlorcyclizinhydrochlorid, Pheniraminmaleat oder Phenindamintartrat; Dekongestionsmittel, beispielsweise Phenylpropanolaminhadrochlorid, Phenylephrinhydrochlorid, Pseudoephidrinhydrochlorid, Pseudoephidrinsulfat, Phenylpropanolaminbitartrat oder Ephedrin; Beta-Rezeptorblocker, beispielsweise Propanolol; Alkoholentwöhnungsmittel, beispielsweise Disulfiram; Antitussiva, beispielsweise Benzocain, Dextrometorphan, Dextrometophanhydrobromid, Noscapin, Carbetapentancitrat oder Chlophedianolhadrochlorid; Fluorergänzungsmittel, beispielsweise Natriumfluorid; Lokalantibiotika, beispielsweise Tetracycline oder Cleocin; Corticosteroidergänzungsmittel, beispielsweise Predinson oder Prednisolon; Mittel gegen Kropfbildung, beispielsweise Colchicin oder Allopurinl; Antiepileptika, beispielsweise Phenytoinnatrium; Mittel gegen Dehydrierung, beispielsweise Elektrolyergänzungsmittel; Antispetika, beispielsweise Cetylpyridiniumchlorid; nicht-steroidale entzündungshemmende Wirkstoffe (Antiphlogistica), beispielsweise Acetaminophen, Ibuprofen, Dexibuprofenlysinat, Naproxen oder deren Salze; gastrointestinale Wirkstoffe, beispielsweise Loperamid oder Famotidin; verschiedene Alkaloide, beispielsweise Codeinphosphat, Codeinsulfat oder Morphin; Ergänzungsmittel für Spurenelemente, beispielsweise Kaliumchlorid, Zinkchlorid, Calciumcarbonate, Magnesiumoxid oder andere Alkalimetall- und Erdalkalimetallsalze; Vitamine; Ionenaustauscherharze, beispielsweise Cholestyramin; Cholesterol- und Lipidsenker; Antiarrhythmetika, beispielsweise N-Acetylprocainamid; oder Expectorantien, beispielsweise Guaifenesin.

Insbesondere sind die folgenden Wirkstoffe zu nennen: Ketoprofen, Ibuprofen, Loperamid, Selegelin, Atipamezole, Nikotin, Chinin, Bruzine, Parazetamol, Dextromethorphan, Coffein und andere Xanthine wie Theophiline und Theobromine, Pyrazolon wie Metamizol, Magnesiumsulfat, Zopliclon oder Zolpidem.

Ferner kommen als Wirkstoffe auch pharmakologisch aktive Substanzen in Betracht, die in den nachfolgend genannten Klassen oder Gruppen enthalten sind:
α-adrenerge Agonisten; β-adrenerge Agonisten; α-adrenerge Blocker; β-adrenerge Blocker; Alkohol-Entwöhnungsmittel; Aldose-Reductase-Inhibitoren; Anabolica; narkotische Analgetica, vorzugsweise Codeine, Morphinderivate; nicht-narkotische Analgetica, vorzugsweise Salicylate und deren Derivate; Androgene; Anaesthetica; Appetitzügler; Anthelmintica (wirksam gegen Cestoden, Nematoden, Onchocerca, Schistosomen oder Trematoden); Anti-Akne-Wirkstoffe;
   Anti-Allergica, Antiamöbica (amöbizide Wirkstoffe); Anti-Androgene; Wirkstoffe gegen Angina pectoris; Antiarrhythmica; anti-arteriosklerotische Wirkstoffe; antiarthritische / antirheumatische Wirkstoffe; antibakterielle Wirkstoffe (Antibiotica), vorzugsweise Aminoglycoside, Amphenicole, Ansamycine, β-Lactame (insbesondere Carbapeneme, Cephalosporine, Cephamycine, Monobactame, Oxacepheme, Penicilline), Lincosamide, Macrolide, Polypeptide, Tetracycline; synthetische antibakterielle Wirkstoffe, vorzugsweise 2,4-Diaminopyrimidine, Nitrofurane, Chinolone und -analoge, Sulfonamide, Sulfone; Anticholinergica; Anticonvulsiva; Antidepressiva, vorzugsweise bizyklische Antidepressiva, Hydrazide, Hydrazine, Pyrrolidone, tetrazyklische Antidepressiva; trizyklische Antidepressiva, polycyclische Imide; antidiabetische Mittel, vorzugsweise Biguanide, Sulfonylharnstoff-Derivate; antidiarrhöische Wirkstoffe; Antidiuretica; Anti-Estrogene; Antimykotica / fungizide Wirkstoffe, vorzugsweise Polyene; synthetische Antimykotica / fungizide Wirkstoffe, vorzugsweise Allylamine, Imidazole, Triazole; Antiglaukom-Wirkstoffe; Antigonadotropine; Wirkstoffe gegen Gicht; Antihistaminica, vorzugsweise Alkylamin-Derivate, Aminoalkyl-Ether, Ethylendiamin-Derivate, Piperazine, trizyklische Verbindungen (insbesondere Phenothiazine); antihyperlipoproteinämische Wirkstoffe (Lipidsenker), vorzugsweise Aryloxyalkansäure-Derivate (insbesondere Clofibrinsäurederivate und - analoge), Gallensäuren-sequestrierende (maskierende) Substanzen, HMG-CoA-Reductase-Inhibitoren, Nicotinsäure-Derivate, Schilddrüsenhormone und Analoge davon; antihypertonische / blutdrucksenkende Wirkstoffe, vorzugsweise Benzothiadiazin-Derivate, N-Carboxyalkyl-(Peptid/Lactam)-Derivate, Guanidin-Derivate, Hydrazine / Phthalazine, Imidazol-Derivate, quaternäre Ammoniumverbindungen, Chinazolin-Derivate, Reserpin-Derivate, Sulfonamid-Derivate; Wirkstoffe gegen Schilddrüsenüberfunktion; Wirkstoffe gegen Hypotonie; Wirkstoffe gegen Schilddrüsen-Unterfunktion; nicht-steroidale entzündungshemmende Wirkstoffe (Antiphlogistica), vorzugsweise Aminoarylcarbonsäure-Derivate, Arylessigsäure-Derivate, Arylbuttersäure-Derivate, Arylcarbonsäure-Derivate, Arylpropionsäure-Derivate, Pyrazole, Pyrazolone, Salicylsäure-Derivate, Thiazincarboxamide; Anti-Malaria-Wirkstoffe, vorzugsweise Chinin und dessen Salze, Säuren und Derivate; Anti-Migräne-Wirkstoffe; Wirkstoffe gegen Übelkeit; antineoplastische Wirkstoffe, vorzugsweise alkylierende Agenzien (insbesondere Alkylsulfonate, Aziridine, Ethylenimine und Methylmelamine, Stickstoffsenfgase, Nitrosoharnstoffe), antibiotische Wirkstoffe, Antimetabolite (insbesondere Folsäure-Analoge, Purin-Analoge, Pyrimidin-Analoge), Enzyme, Interferone, Interleukine; hormonale antineoplastische Wirkstoffe, vorzugsweise Androgene, antiadrenale Wirkstoffe, Anti-Androgene, Anti-Estrogene (insbesondere Aromatase-Inhibitoren); antineoplastische Diätzusätze; Anti-Parkinson-Wirkstoffe; Wirkstoffe gegen Phäochromocytome; Wirkstoffe gegen Pneumocystis; Wirkstoffe zur Behandlung von Prostata-Hypertrophie; Anti-Protozoen-Wirkstoffe, vorzugsweise gegen Leishmania, Trichomonas, Trypanosoma; antipruritische Wirkstoffe; Antipsoriasis-Wirkstoffe; antipsychotische Wirkstoffe, vorzugsweise Butyrophenone, Phenothiazine, Thioxanthene, andere tricyclische Wirkstoffe, 4-Arylpiperazine, 4-Arylpiperidine; antipyretische Wirkstoffe; Mittel gegen Rickettsien; Mittel gegen Seborrhöe; Antiseptica, vorzugsweise Guanidine, Halogene und Halogen-Verbindungen, Nitrofurane, Phenole, Chinoline; antispastische / krampflösende Wirkstoffe; Antithrombotica; Antitussiva; Anti-Ulcus-Wirkstoffe; Uricostatica (Antiurolithica); Antivenenum; antivirale Wirkstoffe, vorzugsweise Purine, Pyrimidinone; Anxiolytica, vorzugsweise Arylpiperazine, Benzodiazepin-Derivate, Carbamate; Benzodiazepin-Antagonisten; Bronchodilatatoren, vorzugsweise Ephedrin-Derivate, quaternäre AmmoniumVerbindungen, Xanthin-Derivate; Calciumkanal-Blocker, vorzugsweise Arylalkylamine, Dihydropyridin-Derivate, Piperazin-Derivate; Calcium-Regulatoren; Cardiotonica; Chelat- bzw. Komplexbildner; Cholecystokinin-Antagonisten; cholelitholytische Wirkstoffe; Choleretica; Cholinergica; Cholinesterase-Inhibitoren; Cholinesterase-Reaktivatoren; ZNS-Stimulantien; Dekongestionsmittel; prophylactische Mittel gegen Zahnkaries; Depigmentierungs-Mittel; Diuretica, vorzugsweise organische Quecksilberverbindungen, Pteridine, Purine, Steroide, Sulfonamid-Derivate, Uracile; Dopaminrezeptor-Agonisten; Wirkstoffe gegen Ectoparasiten; Enzyme, vorzugweise Verdauungsenzyme, Penicillininaktivierende Enzme, proteolytische Enzyme; Enzyminduzierende Wirkstoffe; steroidale und nicht-steroidale Estrogene; Magensekretion-Inhibitoren; Glucocorticoide; Gonaden-stimulierende Wirkstoffe; gonadotrope Hormone; Wachstumshormon-Inhibitoren; Wachstumshormon-Releasing-Faktor; Wachstums-Stimulantien; hämolytische Wirkstoffe; Heparin-Antagonisten; hepatoprotektive Mittel, Wirkstoffe zur Behandlung von Leberkrankheiten; Immunomodulatoren; Immunsupprimierende Wirkstoffe; Ionenaustauscher-Harze; Laktation-stimulierende Hormone; LH-RH-Agonisten; lipotrope Wirkstoffe; Mittel gegen Lupus erythematosus; Mineralocorticoide; Miotica; Monoaminoxidase-Inhibitoren; Mucolytica; Muskelrelaxantien; Narcotica-Antagonisten; neuroprotektive Wirkstoffe; Nootropica; Ophthalmica; Ovarialhormone; Oxytozica; Pepsin-Inhibitoren; Peristaltik-Stimulantien; Progestogene; Prolactin-Inhibitoren; Prostaglandine und -analoge; Protease-Inhibitoren; AtmungsStimulantien; Sklerosierungsmittel; Sedativa / Hypnotica, vorzugsweise acyclische Ureide, Alkohole, Amide, Barbitursäure-Derivate, Benzodiazepin-Derivate, Bromide, Carbamate, Chloral-Derivate, Piperidindione, Chinazolon-Derivate; Thrombolytica; thyreotrope Hormone; Uricosurica; Vasodilatatoren (cerebral); Vasodilatatoren (coronar); Vasodilatatoren (peripher); vasoprotektive Mittel; Vitamine, Vitamin-Vorstufen, Vitamin-Extrakte, Vitamin-Derivate; Wundmittel.

Wirkstoffe mit besonders unangenehmen Geschmack sind antibakterielle Mittel auf Basis der Pyridoncarboxylsäure, wobei 5-Amino-1-cyclopropyl-6,8-Difluor-7-(cis-3,5-dimethyl-1-piperazinyl)-1,4-dihydro-4-oxoquinolon-3-carboxylsäure als besonders unangenehm angesehen wird, Enoxacin, Pipemdicsäure, Ciprofloxacin, Ofloxacin und Pefloxacin, Antiepileptika wie Zonisamid, Makrolidantibiotika wie Erathromycin, Beta-Lactamantibiotika wie Penicilline oder Cephalosporine, psychotrope Wirkstoffe wie Chlorpromazin, Wirkstoffe wie Sulpyrin, oder mittel gegen Geschwüre wie Cimetidin.

Die vorliegende Erfindung umfaßt auch solche Zubereitungen, welche eine Kombination von zwei oder mehr Wirkstoffen enthalten. Eine solche Zubereitung kann in mehrfacher Hinsicht vorteilhaft sein, da sich beliebige, therapeutisch sinnvolle Wirkstoffe in die erfindungsgemäße Zubereitung einarbeiten lassen. Dadurch können mehrere gleichzeitig auftretende Symptome oder Zustände durch eine fixe Wirkstoffkombination in einem einzigen Medikament behandelt werden.

Um eine Wirkstoffaufnahme über die Mundschleimhaut zu unterstützen, ist gemäß einer bevorzugten Ausführungsform der Zusatz von Agenzien vorgesehen, welche die Wirkstoffaufnahme beschleunigen (Permeationsenhancer). Als Permeationsenhancer kommen insbesondere in Betracht: Propandiol, Dexpanthenol, Ölsäure; der/die Permeationsenhancer können beispielsweise aus folgender Gruppe ausgewählt sein: Gesättigte oder ungesättigte Fettsäuren, Kohlenwasserstoffe, geradkettige oder verzweigtkettige Fettalkohole, Dimethylsulfoxid, Propylenglykol, Decanol, Dodecanol, 2-Octyldodecanol, Glycerin, Isopropylidenglycerol, Transcutol (= Diethylenglycol-monoethylether), DEET (= N,N-Diethyl-m-Toluol-amid), Solketal, Ethanol oder andere Alkohole, Menthol und andere ätherische Öle oder Bestandteile ätherischer Öle, Laurinsäurediethanolamid, D-alpha-Tocopherol und Dexpanthenol.

Auch Kombinationen von zwei oder mehreren Enhancersubstanzen lassen sich vorteilhaft einsetzen. Die Wirkstoffaufnahme läßt sich ferner mittels durchblutungsfördernder Stoffe verbessern, welche den erfindungsgemäßen Zubereitungen zugesetzt werden können. Hierzu zählen insbesondere Menthol, Eukalyptol, Ginkgo-Extrakt, Geranium-Öl, Campher, Krauseminzöl, Wacholderbeerenöl und Rosmarin. Diese durchblutungsfördernenden Stoffe können einzeln oder in Kombination, oder auch in Kombination mit einem oder mehreren der vorgenannten Permeationsenhancer-Stoffe eingesetzt werden.

Gemäß einer besonderen Ausführungsform sind die erfindungsgemäßen film- oder oblatenförmigen Arzneizubereitungen zerfallsfähig. Sie können beispielsweise als schnell zerfallende, d. h. innerhalb eines Zeitraums von 1 Sekunde bis 3 Minuten, oder langsam zerfallende, d. h. innerhalb eines Zeitraums von 3 bis 15 Minuten, Darreichungsform ausgelegt sein. Aber auch Darreichungsformen, die lutschbar sind, sind Gegenstand der vorliegenden Erfindung.

Systeme, die mucoadhäsiv sind, aber nicht oder sehr langsam zerfallen bzw. erodieren, müssen nach erfolgter Wirkstofffreisetzung entfernt werden, wobei die Verweildauer des Systems bis zu mehrere Stunden betragen kann.

Der Zerfallsvorgang sollte innerhalb von 15 min im wesentlichen beendet sein, sofern die auf der Schleimhaut haftende Arzneiform während dieser Zeit von einem wässrigen Medium, z. B. einer Körperflüssigkeit, umgeben war. Gemäß bevorzugten Ausführungsformen der Erfindung sind die Arzneiformen so gestaltet, daß sie innerhalb von 3 min und besonders bevorzugt innerhalb von 60 s nach Einbringen in ein wässriges Medium zerfallen.

Die angegebenen Zerfallszeiten beruhen auf der Zerfallszeitenmessung nach Pharm. Eur. 2.9.1 "Zerfallszeiten von Tabletten und Kapseln." Die angegebenen Zerfallszeiten können durch die Verwendung von matrixbildenden Polymeren, welche unterschiedliche Zerfalls- bzw. Löslichkeitscharakteristiken haben, in den genannten Bereichen eingestellt werden. Beispielsweise zerfällt eine Arzneizubereitung auf der Basis von Polyvinylalkohol erheblich schneller als eine HPMC-Arzneizubereitung. Durch Mischen entsprechender Polymerbestandteile läßt sich also die Zerfallszeit justieren. Darüber hinaus sind Sprengmittel bekannt, welche Wasser in die Matrix "ziehen" und diese von innen her aufsprengen. Folglich können auch solche Sprengmittel zwecks Einstellung der Zerfallszeit zugesetzt werden.

Die Matrix der erfindungsgemäßen schnell zerfallenden Darreichungsformen enthält als Grundsubstanzen ein wasserlösliches Polymer, oder Mischungen solcher Polymere. Dabei werden bevorzugt synthetische oder teilsynthetische Polymere oder Biopolymere natürlichen Ursprungs verwendet, die filmbildend und wasserlöslich sind. Besonders geeignet sind Polymere, die vorzugsweise aus der Gruppe ausgewählt sind, welche Cellulosederivate, Polyvinylalkohol (z. B. Mowiol®), Polyacrylate und Polyvinylpyrrolidon umfaßt.

Unter den Cellulosederivaten werden Hydroxypropylmethylcellulose, Carboxymethylcellulose, Natrium-Carboxymethylcellulose (z. B. Walocel), Hydroxyethylcellulose, Hydroxypropylcellulose und Methylcellulose besonders bevorzugt. Ebenfalls bevorzugt sind wasserlösliche Polysaccharide, die pflanzlichen oder mikrobiellen Ursprungs sind, insbesondere Pullulan, Xanthan, Alginate, Dextrane und Pektine. Ferner sind auch Proteine, vorzugsweise Gelatine oder andere gelbildende Proteine, geeignet. Ferner sind Stärke und Stärkederivate; Gelatine (verschiedene Typen); Polyvinylpyrrolidon; Gummi arabicum; Pullulan; Acrylate; Polyethylenoxid, insbesondere die Typen Polyox 10, Polyox 80, Polyox 205, Polyox 301, Polyox 750 (Fa. Union Carbide); Copolymere aus Methylvinyl-Ether und Maleinsäure-Anhydrid (Gantrez-Copolymere, insbesondere die Typen ES, MS, S; Fa. ISP Global Technologies GmbH) geeignet.

Für den Aufbau einer den Wirkstoff langsam freisetzenden Matrix kommen vorzugsweise Polymere zum Einsatz, welche ausgewählt sind aus der Gruppe, die Cellulose-Ether, bevorzugt Ethylcellulose, sowie Polyvinylalkohol, Polyurethan, Polymethacrylate, Polymethylmethacrylate und Derivate und Copolymerisate der vorgenannten Polymere umfaßt.

Die niedrige Löslichkeit oder Unlöslichkeit des Polymerfilms in wäßrigem Milieu, oder auch dessen wasserresistente Ausgestaltung, hat zur Folge, daß die Wirkstoffabgabe nur langsam auf dem Diffusionswege erfolgt, mit - bei geeigneter Formulierung - niedrigem Diffusionskoeffizienten. Dies bewirkt eine langsame Wirkstoffabgabe.

Um die Löslichkeit bzw. die Freisetzungsgeschwindigkeit der langsam freisetzenden Schicht(en) zu verringern, kann die Polymerschicht einer Temperung unterworfen werden. So kann beispielsweise ein hochhydrolysierter Polyvinylalkohol als Basispolymer für die nicht lösliche, langsam freisetzende Schicht eingesetzt werden, wenn dieser durch Temperung unlöslich gemacht wird.

Die Wirkstoffabgabe erfolgt bei den erfindungsgemäßen Zubereitungen auf dem Wege der Permeation durch die orale Mucosa. Voraussetzung hierfür ist, daß die flächige Zubereitung während der Applikationsdauer, d. h. möglichst bis zur erfolgten Auflösung bzw. Zerfall der Zubereitung, in engem Kontakt mit der Mucosa steht. Durch die Auswahl geeigneter Hilfsstoffe läßt sich ein verbesserter Kontakt der erfindungsgemäßen Arzneizubereitung mit der Mundschleimhaut herbeiführen. Deshalb enthält die Arzneizubereitung gemäß einer bevorzugten Ausführungsform der Erfindung einen haftungsvermittelnden Hilfsstoff oder ein Hilfsstoffgemisch, welches der Zubereitung bio- oder mucoadhäsive Eigenschaften verleiht. Von bestimmten pharmazeutisch gebräuchlichen, oral applizierbaren Hilfsstoffen ist bekannt, daß sie schleimhauthaftende Eigenschaften aufweisen. Beispiele für solche mucoadhäsiven Substanzen sind Polyacrylsäure, Carboxymethylcellulose, Hydroxymethylcellulose, Methylcellulose, Traganth, Alginsäure, Gelatine und Gummi arabicum. Darüber hinaus ist von verschiedenen nicht mucoadhäsiven Stoffen bekannt, daß sie in bestimmten Mischungsverhältnissen ebenfalls mucoadhäsive Eigenschaften ausbilden. Ein Beispiel für ein solches Gemisch ist Glycerinmonooleat/Wasser im Verhältnis 84:16 (Engström et al., Pharm. Tech. Eur. 7 [1995], Nr. 2, S. 14-17).

Bei der Verwendung bio- oder mucoadhäsiver Hilfsstoffe ist ein zwei- oder mehrschichtiger Aufbau der Darreichungsform der erfindungsgemäßen Zubereitung zu bevorzugen. Dadurch, daß nur die der Mundschleimhaut zugewandte bzw. mit dieser in Kontakt befindliche Schicht oder Schichten mucoadhäsiv ausgerüstet ist bzw. sind, nicht aber die distal oder außen gelegene Schicht oder Schichten, kann vermieden werden, daß die Zubereitung während der Anwendungsdauer verschiedene Schleimhautpartien miteinander verklebt, was zu erheblichen Mißempfindungen bei der Anwendung führen würde. Bevorzugte Ausführungsformen sind deshalb zwei- oder mehrschichtig aufgebaut, wobei eine der beiden Schichten, bzw. bei mehrschichtigem Aufbau eine der Schichten, bio- oder mucoadhäsive Eigenschaften besitzt. Dieser Aufbau ist bevorzugt bei nicht oder sehr langsam zerfallenden bzw. erodierenden Systemen.

Bei Ausführungsformen, welche neben mucoadhäsiven auch nicht mucoadhäsive Schichten enthalten, werden die letztgenannten vorzugsweise so ausgestaltet, daß sie eine im Vergleich zur bio- oder mucoadhäsiven Schicht geringere Permeabilität für den Wirkstoff besitzt bzw. besitzen. Hierdurch kann vermieden werden, daß Wirkstoff in den Speichel der Mundhöhle freigesetzt wird, was zu Wirkstoffverlusten führen würde.

Die genannten Arzneizubereitungen sind vergleichsweise dichte Gebilde und weisen bevorzugt eine Dichte zwischen 0,3 g/cm³ und 1,7 g/cm³ auf, besonders bevorzugt zwischen 0,5 g/cm³ und 1,5 g/cm³, und am meisten bevorzugt zwischen 0,7 g/cm³ und 1,3 g/cm³.

Die Gesamtdicke der erfindungsgemäßen Zubereitungen beträgt vorzugsweise 5 µm bis 10 mm, bevorzugt 30 µm bis 2 mm und besonders bevorzugt 0,1 mm bis 1 mm. Die Arzneizubereitungen können vorteilhaft runde, ovale, ellipsenförmige, drei-, vier- oder vieleckige Formen aufweisen, sie können aber auch eine beliebig gerundete Form haben.

Die Oberfläche der erfindungsgemäßen Zubereitungen ist üblicherweise glatt; jedoch kann es vorteilhaft sein, die Oberfläche mit Erhebungen und Vertiefungen zu versehen, z. B. in Gestalt von Noppen oder Rillen.

Die Erfindung schließt auch Zubereitungen der genannten Art mit ein, die in Form dünner, fester Schäume vorliegen. Wafer in Form dünner Schäume sind vorteilhaft, da sie auf Grund ihrer großen spezifischen Oberfläche schnell anhaften, andererseits aber auch schnell zerfallen. Die Dichte dieser verfestigten Schäume liegt vorzugsweise zwischen 0,01 g/cm³ und 0,8 g/cm³, besonders bevorzugt zwischen 0,08 g/cm³ und 0,4 g/cm³, und am meisten bevorzugt zwischen 0,1 g/cm³ und 0,3 g/cm³. Bei der Berechnung der Dichte wird das durch den Gesamtkörper des Schaums ausgefüllte oder umhüllte Volumen zugrunde gelegt.

Die genannten Schäume können durch Einleiten und Dispergieren von Gasen mit Hilfe spezieller Schaumaufschlag-Vorrichtungen erzeugt werden, oder durch das Lösen von Gas unter Druck und anschließende Entspannung der Lösung.

Die Matrix der erfindungsgemäßen Arzneizubereitungen weist mindestens ein matrixbildendes Polymer auf. Das oder die matrixbildende(n) Polymer(e) stellt/stellen einen wesentlichen Bestandteil der Matrix dar; der Polymer-Anteil beträgt mindestens 3 Gew.-% und höchstens 98 Gew.-%, vorzugsweise 7 bis 80 Gew.-%, besonders bevorzugt 20 bis 50 Gew.-%, jeweils bezogen auf die gesamte Zubereitung. Die mucoadhäsiven Eigenschaften sowie die Zerfalls-Eigenschaften werden im wesentlichen durch die Art des/der matrixbildenden Polymers/Polymere, sowie die relativen Anteile dieser Polymere in der Zubereitung bestimmt.

Um die Anhaftungstendenz der Darreichungsformen weiter zu vermindern, kann zusätzlich von der Maßnahme Gebrauch gemacht werden, daß die Oberflächen der Darreichungsform uneben oder unregelmäßig geformt sind, vorzugsweise wellenförmig oder reliefartig. Eine solche unregelmäßige Oberflächenstruktur kann beispielsweise durch die in die Polymermatrix eingebrachten blasenförmigen Hohlräume verursacht werden.

Neben den matrixbildenden Polymeren können der Matrix wahlweise Hilfsstoffe zugesetzt werden. Hierfür kommen Füllstoffe (z. B. SiO₂); Farbstoffe und Pigmente (z. B. Chinolingelb oder TiO₂); Sprengmittel, insbesondere Sprengmittel, die Wasser in die Matrix hineinziehen und die Matrix von innen her sprengen (z. B. Aerosil); Emulgatoren (z. B. polyethoxylierte Sorbitanfettsäureester wie TWEEN® oder polyethoxylierte Fettalkohole wie BRIJ®); Weichmacher (z. B. Polyethylenglykol, Glycerin); Süßstoffe (z. B. Aspartam, Saccharin); Konservierungsmittel (z. B. Sorbinsäure und deren Salze) und Aromastoffe in Betracht.

Ferner können als Hilfsstoffe auch Stabilisatoren oder Antioxidantien hinzugefügt werden, wie z. B. Ascorbylpalmitat, Natriumdisulfit, Vitamin E, Vitamin A, Vitamin C; sowohl einzeln als auch in Kombination untereinander, oder in Kombination mit anderen Hilfsstoffen.

Gemäß einer bevorzugten Ausführungsform enthalten die erfindungsgemäßen Zubereitungen mindestens einen Aromastoff und/oder mindestens einen Süßstoff und/oder mindestens einen Weichmacher.

Die Zusammensetzung der erfindungsgemäßen Zubereitungen wird an Hand der nachfolgenden Rezepturen beispielhaft erläutert:

### Beispiel:

| | Beispiel 1 | Beispiel 2 |
|---|---|---|
| Metolose 60 SH 50 | 45,0 | 45,0 |
| Aspartam | 10,0 | 10,0 |
| Mannitol | 10,0 | 10,0 |
| Menthol | 5,0 | 5,0 |
| Aroma | 10,0 | 5,0 |
| Titandioxid | - | 5,0 |
| Natriumhydrogencarbonat | 10,0 | 10,0 |
| Loperamid | 10,0 | 10,0 |

Bei einem Geschmackstest hat sich gezeigt, daß der Zusatz von 10 Gew.-% Natriumhydrogencarbonat, was 2,0 mg/Wafer entspricht, zu einem vollständigen Verschwinden der bitteren Geschmacksempfindung von Loperamid führte.

## Patentansprüche

1. Film- oder oblatenförmige Arzneizubereitung geeignet zur oralen Verabreichung von Wirkstoffen, wobei die Zubereitung eine Matrix aufweist, die aus mindestens einem matrixbildenden Polymer gebildet ist und in welcher mindestens ein bitter schmeckender Wirkstoff, der über die Mundschleimhaut oder im Magen und/oder Darm resorbierbar ist, sowie mindestens eine Kohlendioxid bildende Substanz gelöst oder dispergiert ist, **dadurch gekennzeichnet, dass** die Kohlendioxid bildende Substanz aus der Gruppe ausgewählt ist, die aus Natriumhydrogencarbonat, Natriumcarbonat, Kaliumcarbonat und Kaliumhydrogencarbonat besteht, und nicht in Kombination mit einer Säurekomponente vorliegt, und daß der Wirkstoff aus der Antipyretika, Laxantia, Appetitzügler, Antazida, Antiasthmatika, Antidiuretika, Mittel gegen Blähungen, Migränemittel, Psychopharmaka, Spasmolytika, Sedativa, Antihyperkinetika, Tranquilizer, Antihistaminika, Dekongestionsmittel, Beta-Rezeptorblocker, Alkoholentwöhnungsmittel, Fluorergänzungsmittel, Lokalantibiotika, Corticosteroidergänzungsmittel, Mittel gegen Kropfbildung, Antiepileptika, Mittel gegen Dehydrierung, Antiseptika, Acetaminophen, Naproxen, gastrointestinale Wirkstoffe, Alkaloide, Ergänzungsmittel für Spurenelemente, Cholesterol- und Lipidsenker, Antiarrhythmika, Expectorantien, Xanthine, Pyrazolone, α-adrenerge Agonisten, β-adrenerge Agonisten, α-adrenerge Blocker, Aldose-Reductase-Inhibitoren, Anabolica, narkotische Analgetika, Androgene, Anthelminthica, Anti-Akne-Wirkstoffe, Antiallergika, amöbizide Wirkstoffe, Anti-Androgene, Wirkstoff gegen Angina pectoris, anti-arteriosklerotische Wirkstoffe, Antibiotika, Anticholinergica, Anticonvulsiva, Antidepressiva, antidiabetische Mittel, antidiarrhöische Wirkstoffe, Antidiuretica, Anti-Estrogene, Antimykotica, Antiglaukom-Wirkstoffe, Antigonadotropine, Wirkstoffe gegen Gicht, Gallensäuresequestrierende Substanzen, Nicotinsäure-Derivate, Schilddrüsenhormone, antihypertonische Wirkstoffe, Wirkstoffe gegen Schilddrüsenüberfunktion, Wirkstoffe gegen Schilddrüsenunterfunktion Wirkstoffe gegen Hypotonie, Antimalaria-Wirkstoffe, Wirkstoffe gegen Übelkeit, antineoplastische Wirkstoffe, Anti-Parkinson-Wirkstoffe, Wirkstoffe zur Behandlung von Prostata-Hypertrophie, Antiprotozoen-Wirkstoffe, antipruritische Wirkstoffe, Antipsoriasis-Wirkstoffe, antipsychotische Wirkstoffe, Antithrombotica, Antitussiva, Anti-Ulcus-Wirkstoffe, Uricostatica, antivirale Wirkstoffe, Bronchodilatatoren, Calciumkanal-Blocker, Cardiotonica, Cholecystokinin-Antagonisten, cholelitholytische Wirkstoffe, Choleretica, Cholinergica, Cholinesterase-Inhibitoren, Cholinesterase-Reaktivatoren, ZNS-Stimulantien, Diuretica, Dopaminrezeptor-Agonisten, Wirkstoffe gegen Ectoparasiten, Enzyme, enzyminduzierende Wirkstoffe, steroidale und nichtsteroidale Estrogene, Magensekretion-Inhibitoren, Glucocorticoide, gonadenstimulierende Wirkstoffe, gonadotrope Hormone, Wachstumshormon-Inhibitoren, Wachstumshormon-Releasing-Factor, Wachstums-Stimulantien, hämolytische Wirkstoffe, Heparin-Antagonisten, hepatoprotektive Mittel, Mittel zur Behandlung von Leberkrankheiten, Immunomodulatoren, immunsupprimierende Wirkstoffe, Laktation-stimulierende Hormone, LH-RH-Agonisten, lipotrope Substanzen, Mittel gegen Lupus erythematosus, Mineralocorticoide, Miotica, Mucolytica, Muskelrelaxantien, Narcotica-Antagonisten, neuroprotektive Wirkstoffe, Nootropica, Ophthalmica, Ovarialhormone, Oxytozica, Pepsin-Inhibitoren, Peristaltik-Stimulantien, Progestogene, Prolactin-Inhibitoren, Prostaglandine, Protease-Inhibitoren, Atmungsstimulantien, Sedativa, Hypnotica, Thrombolytica, Uricosurica, Vasodilatatoren, vasoprotektive Mittel, Vitamine, Vitamin-Vorstufen und Vitamin-Extrakte umfassenden Gruppe ausgewählt ist.

2. Film- oder oblatenförmige Arzneizubereitung nach Anspruch 1, **dadurch gekennzeichnet, daß** die Arzneizubereitung zur Verabreichung von Wirkstoff(en) über die Mundschleimhaut geeignet ist.

3. Film- oder oblatenförmige Arzneizubereitung nach einem der vorhergehenden Ansprüche, **dadurch gekennzeichnet, daß** der Kohlendioxidbildner in einer Menge von 2 bis 50 Gew.-%, bevorzugt von 5 bis 30 Gew.-%, und besonders bevorzugt von 7 bis 20 Gew.-%, bezogen auf die Arzneizubereitung, in der Arzneizubereitung enthalten ist.

4. Film- oder oblatenförmige Arzneizubereitung nach einem der vorhergehenden Ansprüche, **dadurch gekennzeichnet, daß** sie mindestens einen Permeationsenhancer und/oder mindestens einen durchblutungsfördernden Stoff enthält.

5. Film- oder oblatenförmige Arzneizubereitung nach Anspruch 4, **dadurch gekennzeichnet, daß** der Permeationsenhancer aus der Gruppe ausgewählt ist, die gesättigte oder ungesättigte Fettsäuren, Kohlenwasserstoffe, geradkettige oder verzweigtkettige Fettalkohole, Dimethylsulfoxid, Propylenglykol, Decanol, Dodecanol, 2-Octyldodecanol, Glycerin, Isopropylidenglycerol, Transcutol (= Diethylenglycolmonoethylether), DEET (= N,N-Diethyl-m-Toluolamid), Solketal, Ethanol oder andere Alkohole, Menthol und andere ätherische Öle oder Bestandteile ätherischer Öle, Laurinsäurediethanolamid, D-alpha-Tocopherol und Dexpanthenol umfasst.

6. Film- oder oblatenförmige Arzneizubereitung nach Anspruch 4, **dadurch gekennzeichnet, daß** der durchblutungsfördernde Stoff aus der Gruppe ausgewählt ist, die Menthol, Eukalyptol, Ginkgo-Extrakt, Geranium-Öl, Campher, Krauseminzöl, Wacholderbeerenöl und Rosmarin umfasst.

7. Film- oder oblatenförmige Arzneizubereitung nach einem der vorhergehenden Ansprüche, **dadurch gekennzeichnet, daß** sie innerhalb von 15 min, bevorzugt innerhalb von 3 min und besonders bevorzugt innerhalb von 60 Sekunden nach Einbringen in ein wässriges Medium zerfällt.

8. Film- oder oblatenförmige Arzneizubereitung nach einem der vorhergehenden Ansprüche, **dadurch gekennzeichnet, daß** das/die matrixbildende(n) Polymer(e) aus der Polyvinylalkohol, Cellulosederivate, Stärke und Stärkederivate, Gelatine, Polyvinylpyrrolidon, Gummi arabicum, Pullulan, Acrylate, Polyethylenoxid und Copolymere aus Methylvinyl-Ether und Maleinsäure-Anhydrid umfassenden Gruppe ausgewählt ist/sind, wobei die Gruppe der Cellulosederivate bevorzugt Hydroxypropylmethylcellulose, Hydroxypropylcellulose, Natrium-Carboxymethylcellulose, Methylcellulose, Hydroxyethylcellulose und Hydroxypropylethylcellulose umfaßt.

9. Film- oder oblatenförmige Arzneizubereitung nach einem der Ansprüche 1 bis 6, **dadurch gekennzeichnet, daß** das/die matrixbildende(n) Polymer(e) aus der Gruppe ausgewählt ist, die Cellulose-Ether, bevorzugt Ethylcellulose, sowie Polyvinylalkohol, Polyurethan, Polymethacrylate, Polymethylmethacrylate und Derivate und Copolymerisate der vorgenannten Polymere umfaßt.

10. Film- oder oblatenförmige Arzneizubereitung nach einem der vorheregehenden Ansprüche, **dadurch gekennzeichnet, daß** die Arzneizubereitung einen Hilfsstoff enthält, welcher der Zubereitung mucoadhäsive Eigenschaften verleiht.

11. Film- oder oblatenförmige Arzneizubereitung nach Anspruch 10, **dadurch gekennzeichnet, daß** der Hilfsstoff aus der Gruppe ausgewählt ist, die Polyacrylsäure, Carboxymethylcellulose, Hydroxymethylcellulose, Methylcellulose, Traganth, Alginsäure, Gelatine und Gummi arabicum oder ein Gemisch davon umfaßt.

12. Film- oder oblatenförmige Arzneizubereitung nach Anspruch 10, **dadurch gekennzeichnet, daß** die Arzneiform zwei- oder mehrschichtig aufgebaut ist, wobei nur die der Mundschleimhaut zugewandte bzw. mit dieser in Kontakt befindliche Schicht oder Schichten mucoadhäsiv ausgerüstet ist bzw. sind.

13. Film- oder oblatenförmige Arzneizubereitung nach Anspruch 12, **dadurch gekennzeichnet, daß** die nicht-mucoadhäsiven Schichten eine geringere Permeabilität für den Wirkstoff bzw. die Wirkstoffe aufweisen.

14. Film- oder oblatenförmige Arzneizubereitung nach einem der vorhergehenden Ansprüche, **dadurch gekennzeichnet, daß** sie flächenförmig ist, wobei die Dichte dieser flächenförmigen Zubereitung vorzugsweise zwischen 0,3 g/cm³ und 1,7 g/cm³, besonders bevorzugt zwischen 0,5 g/cm³ und 1,5 g/cm³, und am meisten bevorzugt zwischen 0,7 g/cm³ und 1,3 g/cm³ liegt.

15. Film- oder oblatenförmige Arzneizubereitung nach einem der vorhergehenden Ansprüche, **dadurch gekennzeichnet, daß** ihre Gesamtdicke 5 µm bis 10 mm, bevorzugt 30 µm bis 2 mm und besonders bevorzugt 0,1 mm bis 1 mm beträgt.

16. Film- oder oblatenförmige Arzneizubereitung nach einem der vorhergehenden Ansprüche, **dadurch gekennzeichnet, daß** sie eine runde oder ellipsenförmige oder ovale Form, oder eine drei-, vier- oder vieleckige Form, oder eine unregelmäßig gerundete Form aufweist.

17. Film- oder oblatenförmige Arzneizubereitung nach einem der vorhergehenden Ansprüche, **dadurch gekennzeichnet, daß** sie als fester Schaum vorliegt, wobei die Dichte dieses verfestigten Schaumes vorzugsweise zwischen 0,01 g/cm³ und 0,8 g/cm³, besonders bevorzugt zwischen 0,08 g/cm³ und 0,4 g/cm³, und am meisten bevorzugt zwischen 0,1 g/cm³ und 0,3 g/cm³ liegt.

18. Film- oder oblatenförmige Arzneizubereitung nach einem der vorhergehenden Ansprüche, **dadurch gekennzeichnet, daß** der Polymer-Anteil der Matrix mindestens 3 Gew.-% und höchstens 98 Gew.-%, vorzugsweise 7 bis 80 Gew.-%, besonders bevorzugt 20 bis 50 Gew.-%, jeweils bezogen auf die gesamte Zubereitung, beträgt.

19. Film- oder oblatenförmige Arzneizubereitung nach einem der vorhergehenden Ansprüche, **dadurch gekennzeichnet, daß** sie mindestens einen Hilfsstoff enthält, wobei der/die Hilfsstoffe aus der Füllstoffe, Farbstoffe, Sprengmittel, Emulgatoren, Weichmacher, Süßstoffe, Konservierungsmittel, Stabilisatoren, Antioxidantien und Aromastoffe umfassenden Gruppe ausgewählt ist/sind.

20. Film- oder oblatenförmige Arzneizubereitung nach einem der vorhergehenden Ansprüche, **dadurch gekennzeichnet, daß** sie mindestens einen Aromastoff und/oder mindestens einen Süßstoff und/oder mindestens einen Weichmacher enthält.

21. Film- oder oblatenförmige Arzneizubereitung nach einem der vorhergehenden Ansprüche, **dadurch gekennzeichnet, daß** der Wirkstoff aus der Acetaminophen, Aspirin, Phenolphthaleindioctylnatriumsulfosuccinat, Phenylpropanolamin, Coffein, Theophyllin, Diphenoxylathydrochlorid, Simethicon, Ergotamintartrat, Haloperidol, Phenobarbitol, Methyldopa, Methylphenidat, Benzodiazepine, Phenothiazine, Astermizol, Pheniraminmaleat, Chlorpheniraminmaleat, Brompheniraminmaleat, Pyridaminmaleat, Doxylaminsuccinat, Phenyltoloxamincitrat, Chlorcyclizinhydrochlorid, Phenylpropanolaminhydrochlorid, Phenylpropanolaminbitartrat, Phenylephrinhydrochlorid, Pseudoephedrinhydrochlorid, Pseudoephedrinsulfat, Ephedrin, Propranolol, Disulfiram, Dextrometorphan, Dextrometorphanhydrochlorid, Noscapin, Carbepentancitrat, Chlophedianolhydrochlorid, Tetracycline, Cleocin, Prednison, Prednisolon, Colchicin, Allopurinol, Phenytoin-Natrium, Cetylpyridiniumchlorid, Naproxen, Loperamid, Famotidin, Codeinphosphat, Codeinsulfat, Morphin, Kaliumchlorid, Zinkchlorid, Cholestyramin, N-Acetylprocainamid, Guaifenesin, Selegelin, Atipamezol, Nicotin, Chinin, Bruzin, Dextromethorphan, Coffein, Theophyllin, Theobromin, Metamizol, Magnesiumsulfat, Zopliclon, Zolpidem, antibakterielle Mittel auf Basis der Pyridoncarboxylsäure, Enoxacin, Pipemidsäure, Ciproflaxin, Ofloxacin, Pefloxacin, Zonisamid, Erythromycin, Penicilline, Cephalosporine, Chlorpromazin, Sulpyrin und Cimetidin umfassenden Gruppe ausgewählt ist.

22. Verwendung einer Kohlendioxid-bildenden Substanz, oder einer Kombination solcher Substanzen, als geschmacksmaskierender Zusatz in einer film- oder oblatenförmigen, zur oralen Verabreichung geeigneten Arzneizubereitung, welche eine Matrix aufweist, die aus mindestens einem matrixbildenden Polymer gebildet ist und in welcher mindestens ein bitter schmeckender Wirkstoff gelöst oder dispergiert ist, der über die Mundschleimhaut oder im Magen und/oder Darm resorbierbar ist, wobei die Kohlendioxid-bildende Substanz aus der Gruppe ausgewählt ist, die aus Natriumhydrogencarbonat, Natriumcarbonat, Kaliumcarbonat und Kaliumhydrogencarbonat besteht, und nicht in Kombination mit einer Säurekomponente vorliegt, und wobei der Wirkstoff aus der Antipyretika, Laxantia, Appetitzügler, Antazida, Antiasthmatika, Antidiuretika, Mittel gegen Blähungen, Migränemittel, Psychopharmaka, Spasmolytika, Sedativa, Antihyperkinetika, Tranquilizer, Antihistaminika, Dekongestionsmittel, Beta-Rezeptorblocker, Alkoholentwöhnungsmittel, Fluorergänzungsmittel, Lokalantibiotika, Corticosteroidergänzungsmittel, Mittel gegen Kropfbildung, Antiepileptika, Mittel gegen Dehydrierung, Antiseptika, Acetaminophen, Naproxen, gastrointestinale Wirkstoffe, Alkaloide, Ergänzungsmittel für Spurenelemente, Cholesterol- und Lipidsenker, Antiarrhythmika, Expectorantien, Xanthine, Pyrazolone, α-adrenerge Agonisten, β-adrenerge Agonisten, α-adrenerge Blocker, Aldose-Reductase-Inhibitoren, Anabolica, narkotische Analgetika, Androgene, Anthelminthica, Anti-Akne-Wirkstoffe, Antiallergika, amöbizide Wirkstoffe, Anti-Androgene, Wirkstoff gegen Angina pectoris, anti-arteriosklerotische Wirkstoffe, Antibiotika, Anticholinergica, Anticonvulsiva, Antidepressiva, antidiabetische Mittel, antidiarrhöische Wirkstoffe, Antidiuretica, Anti-Estrogene, Antimykotica, Antiglaukom-Wirkstoffe, Antigonadotropine, Wirkstoffe gegen Gicht, Gallensäuresequestrierende Substanzen, Nicotinsäure-Derivate, Schilddrüsenhormone, antihypertonische Wirkstoffe, Wirkstoffe gegen Schilddrüsenüberfunktion, Wirkstoffe gegen Schilddrüsenunterfunktion Wirkstoffe gegen Hypotonie, Antimalaria-Wirkstoffe, Wirkstoffe gegen Übelkeit, antineoplastische Wirkstoffe, Anti-Parkinson-Wirkstoffe, Wirkstoffe zur Behandlung von Prostata-Hypertrophie, Antiprotozoen-Wirkstoffe, antipruritische Wirkstoffe, Antipsoriasis-Wirkstoffe, antipsychotische Wirkstoffe, Antithrombotica, Antitussiva, Anti-Ulcus-Wirkstoffe, Uricostatica, antivirale Wirkstoffe, Bronchodilatatoren, Calciumkanal-Blocker, Cardiotonica, Cholecystokinin-Antagonisten, cholelitholytische Wirkstoffe, Choleretica, Cholinergica, Cholinesterase-Inhibitoren, Cholinesterase-Reaktivatoren, ZNS-Stimulantien, Diuretica, Dopaminrezeptor-Agonisten, Wirkstoffe gegen Ectoparasiten, Enzyme, enzyminduzierende Wirkstoffe, steroidale und nichtsteroidale Estrogene, Magensekretion-Inhibitoren, Glucocorticoide, gonadenstimulierende Wirkstoffe, gonadotrope Hormone, Wachstumshormon-Inhibitoren, Wachstumshormon-Releasing-Factor, Wachstums-Stimulantien, hämolytische Wirkstoffe, Heparin-Antagonisten, hepatoprotektive Mittel, Mittel zur Behandlung von Leberkrankheiten, Immunomodulatoren, immunsupprimierende Wirkstoffe, Laktation-stimulierende Hormone, LH-RH-Agonisten, lipotrope Substanzen, Mittel gegen Lupus erythematosus, Mineralocorticoide, Miotica, Mucolytica, Muskelrelaxantien, Narcotica-Antagonisten, neuroprotektive Wirkstoffe, Nootropica, Ophthalmica, Ovarialhormone, Oxytozica, Pepsin-Inhibitoren, Peristaltik-Stimulantien, Progestogene, Prolactin-Inhibitoren, Prostaglandine, Protease-Inhibitoren, Atmungsstimulantien, Sedativa, Hypnotica, Thrombolytica, Uricosurica, Vasodilatatoren, vasoprotektive Mittel, Vitamine, Vitamin-Vorstufen und Vitamin-Extrakte umfassenden Gruppe ausgewählt ist.

## Claims

1. Film-like or wafer-like medicinal preparation suitable for oral administration of active substances, wherein the preparation contains a matrix composed of at least one matrix-forming polymer and in which at least one bitter-tasting active substance that is absorbable via the oral mucosa or in the stomach and/or intestine and at least one carbon dioxide-forming substance are dissolved or dispersed, **characterized in that** the carbon dioxide-forming substance is selected from the group comprising sodium hydrogencarbonate, sodium carbonate, potassium carbonate and potassium hydrogencarbonate and is not present in combination with an acid component, and wherein the active substance is selected from the group composed of the antipyretics, laxatives, appetite suppressants, antacids, antiasthmatics, antidiuretics, antiflatulents, migraine agents, psychopharmacological agents, spasmolytics, sedatives, antihyperkinetics, tranquilizers, antihistamines, decongestants, β-receptor blockers, agents for alcohol withdrawal, fluorine supplements, local antibiotics, corticosteroid supplements, agents against goiter formation, antiepileptics, agents against dehydration, antiseptics, acetaminophen, naproxen, gastrointestinal agents, alkaloids, trace element supplements, cholesterol- and lipid-reducing agents, antiarrhythmics, expectorants, xanthines, pyrazolones, α-adrenergic agonists, β-adrenergic agonists, α-adrenergic blockers, aldose reductase inhibitors, anabolics, narcotic analgesics, androgens, anthelminthics, antiacne agents, antiallergics, amoebicidal agents, antiandrogens, agents against angina pectoris, antiatherosclerotic agents, antibiotics, anticholinergics, anticonvulsants, antidepressants, antidiabetic agents, antidiarrheal agents, antidiuretics, antiestrogens, antimycotics, antiglaucoma agents, antigonadotropins, antigout agents, bile acid sequestrants, nicotinic acid derivatives, thyroid hormones, antihypertensive agents, antihyperthyroid agents, antihypothyroid agents, antihypotensive agents, antimalaria agents, antiemetic agents, antineoplastic agents, antiparkinson agents, agents for the treatment of prostatic hypertrophy, antiprotozoal agents, antipruritic agents, antipsoriatic agents, antipsychotic agents, antithrombotics, antitussives, antiulcer agents, uricostatics, antiviral agents, bronchodilators, calcium channel blockers, cardiotonics, cholecystokinin antagonists, cholelitholytic agents, choleretics, cholinergics, cholinesterase inhibitors, cholinesterase reactivators, CNS stimulants, diuretics, dopamine receptor agonists, antiectoparasitic agents, enzymes, enzyme-inducing agents, steroidal and nonsteroidal estrogens, gastric secretion inhibitors, glucocorticoids, gonad-stimulating agents, gonadotropic hormones, growth hormone inhibitors, growth hormone releasing factors, growth stimulants, hemolytic agents, heparin antagonists, hepatoprotective agents, agents for the treatment of liver diseases, immunomodulators, immunosuppressive agents, lactation-stimulating hormones, LH-RH agonists, lipotropic agents, agents against lupus erythematosus, mineralocorticoids, miotics, mucolytics, muscle relaxants, narcotic antagonists, neuroprotective agents, nootropics, ophthalmics, ovarian hormones, oxytocic agents, pepsin inhibitors, peristaltic stimulants, progestogens, prolactin inhibitors, prostaglandins, protease inhibitors, respiratory stimulants, sedatives, hypnotics, thrombolytics, uricosuric agents, vasodilators, vasoprotective agents, vitamins, vitamin precursors and vitamin extracts.

2. Film-like or wafer-like medicinal preparation according to Claim 1, **characterized in that** the medicinal preparation is suitable for the administration of (an) active ingredient(s) via the oral mucosa.

3. Film-like or wafer-like medicinal preparation according to one of the preceding claims, **characterized in that** the carbon dioxide-forming substance is contained in the medicinal preparation in an amount of 2 to 50 wt.%, preferably 5 to 30 wt.% and particularly preferably 7 to 20 wt.% based on the medicinal preparation.

4. Film-like or wafer-like medicinal preparation according to one of the preceding claims, **characterized by** containing at least one permeation enhancer and/or at least one blood flow-stimulating substance.

5. Film-like or wafer-like medicinal preparation according to Claim 4, **characterized in that** the permeation enhancer is selected from the group comprising saturated or unsaturated fatty acids, hydrocarbons, linear or branched fatty alcohols, dimethyl sulfoxide, propylene glycol, decanol, dodecanol, 2-octyldodecanol, glycerol, isopropylidene glycerol, transcutol (= diethylene glycol monoethyl ether), DEET (= n,n-diethyl-m-toluolamide), solketal, ethanol or other alcohols, menthol and other essential or components of essential oils, lauric acid diethanolamide, D-α-tocopherol and dexpanthenol.

6. Film-like or wafer-like medicinal preparation according to Claim 4, **characterized in that** the blood flow-stimulating substance is selected from the group comprising menthol, eucalyptol, ginkgo extract, geranium oil, camphor, spearmint oil, juniper berry oil and rosemary.

7. Film-like or wafer-like medicinal preparation according to one of the preceding claims, **characterized by** decomposing within 15 min, preferably within 3 min and particularly preferably within 60 sec after placement in an aqueous medium.

8. Film-like or wafer-like medicinal preparation according to one of the preceding claims, **characterized in that** the matrix-forming polymer(s) is/are selected from the group comprising polyvinyl alcohol, cellulose derivatives, starch and starch derivatives, gelatin, polyvinylpyrrolidone, gum arabic, pullulan, acrylate, polyethylene oxide, and methyl vinyl ether and maleic acid anhydride copolymers, wherein the group of the cellulose derivatives preferably comprises hydroxypropyl methylcellulose, hydroxypropyl cellulose, sodium carboxymethyl cellulose, methyl cellulose, hydroxyethyl cellulose and hydroxypropyl ethylcellulose.

9. Film-like or wafer-like medicinal preparation according to one of Claims 1 to 6, **characterized in that** the matrix-forming polymer(s) is/are selected from the group comprising cellulose ethers, preferably ethyl cellulose, and polyvinyl alcohol, polyurethane, polymethacrylates, polymethyl methacrylates and derivatives and copolymers of the above-mentioned polymers.

10. Film-like or wafer-like medicinal preparation according to one of the preceding claims, **characterized in that** the medicinal preparation contains an excipient that imparts mucoadhesive properties to the preparation.

11. Film-like or wafer-like medicinal preparation according to Claim 10, **characterized in that** the excipient is selected from the group comprising polyacrylic acid, carboxymethyl cellulose, hydroxymethyl cellulose, methyl cellulose, tragacanth, alginic acid, gelatin and gum arabic or a mixture thereof.

12. Film-like or wafer-like medicinal preparation according to Claim 10, **characterized in that** the dosage form has a double-layer or multilayer structure, wherein only the layer or layers facing the oral mucosa or in contact therewith is/are provided with mucoadhesive properties.

13. Film-like or wafer-like medicinal preparation according to Claim 12, **characterized in that** the non-mucoadhesive layers show lower permeability for the active substance or substances.

14. Film-like or wafer-like medicinal preparation according to one of the preceding claims, **characterized by** being sheet-like, wherein the density of this sheet-like preparation is preferably between 0.3 g/cm³ and 1.7 g/cm³, particularly preferably between 0.5 g/cm³ and 1.5 g/cm³ and most preferably between 0.7 g/cm³ and 1.3 g/cm³.

15. Film-like or wafer-like medicinal preparation according to one of the preceding claims, **characterized in that** the total thickness thereof is 5 µm to 10 mm, preferably 30 µm to 2 mm and particularly preferably 0.1 mm to 1 mm.

16. Film-like or wafer-like medicinal preparation according to one of the preceding claims, **characterized by** having a round, elliptical, or oval shape, a triangular, square, or polygonal shape, or an irregularly rounded shape.

17. Film-like or wafer-like medicinal preparation according to one of the preceding claims, **characterized by** being in the form of a solid foam, wherein the density of said solidified foam is preferably between 0.01 g/cm³ and 0.8 g/cm³, particularly preferably between 0.08 g/cm³ and 0.4 g/cm³ and most preferably between 0.1 g/cm³ and 0.3 g/cm³.

18. Film-like or wafer-like medicinal preparation according to one of the preceding claims, **characterized in that** the polymer content of the matrix is at least 3 wt.% and at most 98 wt.%, preferably 7 to 80 wt.% and particularly preferably 20 to 50 wt.%, based in each case on the entire preparation.

19. Film-like or wafer-like medicinal preparation according to one of the preceding claims, **characterized by** containing at least one excipient, wherein the excipient(s) is/are selected from the group comprising fillers, dyes, disintegrants, emulsifiers, softeners, sweeteners, preservatives, stabilizers, antioxidants and flavours.

20. Film-like or wafer-like medicinal preparation according to one of the preceding claims, **characterized by** containing at least one flavour and/or at least one sweetener and/or at least one softener.

21. Film-like or wafer-like medicinal preparation according to one of the preceding claims, **characterized in that** the active substance is selected from the group comprising acetaminophen, aspirin, phenolphthalein dioctyl sodium sulfosuccinate, phenyl propanolamine, caffeine, theophylline, diphenoxylate hydrochloride, simethicone, ergotamine tartrate, haloperidol, phenobarbital, methyldopa, methylphenidate, benzodiazepines, phenothiazines, astemizole, pheniramine maleate, chlorpheniramine maleate, brompheniramine maleate, pyridamine maleate, doxylamine succinate, phenyltoloxamine citrate, chlorcyclizine hydrochloride, phenylpropanolamine hydrochloride, phenylpropanolamine bitartrate, phenylephrine hydrochloride, pseudoephedrine hydrochloride, pseudoephedrine sulfate, ephedrine, propranolol, disulfiram, dextromethorphan, dextromethorphan hydrochloride, noscapine, carbetapentane citrate, chlophedianol hydrochloride, tetracyclines, Cleocin, prednisone, prednisolone, colchicine, allopurinol, phenytoin sodium, cetylpyridinium chloride, naproxen, loperamide, famotidine, codeine phosphate, codeine sulfate, morphine, potassium chloride, zinc chloride, cholestyramine, N-acetylprocainamide, guaifenesin, selegiline, atipamezole, nicotine, quinine, brucine, dextromethorphan, caffeine, theophylline, theobromine, metamizole, magnesium sulfate, zopiclone, zolpidem, pyridonecarboxylic acid-based antibacterial agents, enoxacin, pipemidic acid, ciprofloxacin, ofloxacin, pefloxacin, zonisamide, erythromycin, penicillin, cephalosporin, chlorpromazine, sulpyrine and cimetidine.

22. Use of a carbon dioxide-forming substance or a combination of such substances as a taste-masking additive in a film-like or wafer-like medicinal preparation suitable for oral administration that has a matrix composed of at least one matrix-forming polymer and in which at least one bitter-tasting active substance is dissolved or dispersed, which is absorbable via the oral mucosa or in the stomach and/or intestine, wherein the carbon dioxide-forming substance is selected from the group comprising sodium hydrogencarbonate, sodium carbonate, potassium carbonate and potassium hydrogencarbonate, and is not present in combination with an acid component, and wherein the active substance is selected from the group comprising antipyretics, laxatives, appetite suppressants, antacids, antiasthmatics, antidiuretics, antiflatulents, migraine agents, psychopharmacological agents, spasmolytics, sedatives, antihyperkinetics, tranquilizers, antihistamines, decongestants, β-receptor blockers, agents for withdrawal from alcohol, fluorine supplements, local antibiotics, corticosteroid supplements, agents against goiter formation, antiepileptics, agents against dehydration, antiseptics, acetaminophen, naproxen, gastrointestinal agents, alkaloids, trace element supplements, cholesterol- and lipid-reducing agents, antiarrhythmics, expectorants, xanthines, pyrazolones, α-adrenergic agonists, β-adrenergic agonists, α-adrenergic blockers, aldose reductase inhibitors, anabolics, narcotic analgesics, androgens, anthelminthics, antiacne agents, antiallergics, amoebicidal agents, antiandrogens, agents against angina pectoris, antiatherosclerotic agents, antibiotics, anticholinergics, anticonvulsants, antidepressants, antidiabetic agents, antidiarrheal agents, antidiuretics, antiestrogens, antimycotics, antiglaucoma agents, antigonadotropins, antigout agents, bile acid sequestrants, nicotinic acid derivatives, thyroid hormones, antihypertensive agents, antihyperthyroid agents, antihypothyroid agents, antihypotensive agents, antimalaria agents, antiemetic agents, antineoplastic agents, antiparkinson agents, agents for the treatment of prostatic hypertrophy, antiprotozoal agents, antipruritic agents, antipsoriatic agents, antipsychotic agents, antithrombotics, antitussives, antiulcer agents, uricostatics, antiviral agents, bronchodilators, calcium channel blockers, cardiotonics, cholecystokinin antagonists, cholelitholytic agents, choleretics, cholinergics, cholinesterase inhibitors, cholinesterase reactivators, CNS stimulants, diuretics, dopamine receptor agonists, antiectoparasitic agents, enzymes, enzyme-inducing agents, steroidal and nonsteroidal estrogens, gastric secretion inhibitors, glucocorticoids, gonad-stimulating agents, gonadotropic hormones, growth hormone inhibitors, growth hormone releasing factors, growth stimulants, hemolytic agents, heparin antagonists, hepatoprotective agents, agents for the treatment of liver diseases, immunomodulators, immunosuppressive agents, lactation-stimulating hormones, LH-RH agonists, lipotropic agents, agents against lupus erythematosus, mineralocorticoids, miotics, mucolytics, muscle relaxants, narcotics antagonists, neuroprotective agents, nootropics, ophthalmics, ovarian hormones, oxytocic agents, pepsin inhibitors, peristaltic stimulants, progestogens, prolactin inhibitors, prostaglandins, protease inhibitors, respiratory stimulants, sedatives, hypnotics, thrombolytics, uricosuric agents, vasodilators, vasoprotective agents, vitamins, vitamin precursors and vitamin extracts.

## Revendications

1. Préparation médicamenteuse sous la forme d'un film ou d'un cachet, appropriée pour l'administration orale d'agents actifs, la préparation comprenant une matrice qui est formée par au moins un polymère formant une matrice et dans laquelle au moins un agent actif à goût amer, qui est résorbable par le biais de la muqueuse de la bouche ou dans l'estomac et/ou dans l'intestin, ainsi qu'au moins une substance formant du dioxyde de carbone, sont dissoutes ou dispersées, **caractérisée en ce que** la substance formant du dioxyde de carbone est choisie dans le groupe constitué par l'hydrogénocarbonate de sodium, le carbonate de sodium, le carbonate de potassium et l'hydrogénocarbonate de potassium, et ne se présente pas en combinaison avec un composant acide, et **en ce que** l'agent actif est choisi dans le groupe comprenant les antipyrétiques, les laxatifs, les anorexigènes, les antacides, les antiasthmatiques, les antidiurétiques, les agents contre les flatulences, les agents contre la migraine, les agents psychopharmaceutiques, les spasmolytiques, les sédatifs, les antihyperkinétiques, les tranquillisants, les antihistaminiques, les agents décongestionnants, les bloquants des récepteurs bêta, les agents de désaccoutumance de l'alcool, les compléments de fluor, les antibiotiques locaux, les compléments corticostéroïdes, les agents contre la formation de goitres, les antiépileptiques, les agents contre la déshydratation, les antiseptiques, l'acétaminophène, le naproxène, les agents actifs gastro-intestinaux, les alcaloïdes, les compléments d'oligoéléments, les réducteurs de cholestérol et de lipides, les agents antiarrhythmiques, les expectorants, les xanthines, les pyrazolones, les agonistes α-adrénergiques, les agonistes β-adrénergiques, les bloquants α-adrénergiques, les inhibiteurs d'aldose-réductase, les anaboliques, les analgésiques narcotiques, les androgènes, les anthelminthiques, les agents actifs anti-acné, les antiallergiques, les agents actifs amibicides, les anti-androgènes, les agents actifs contre l'angine de poitrine, les agents actifs anti-artériosclérotiques, les antibiotiques, les anticholinergiques, les anticonvulsifs, les antidépresseurs, les agents antidiabétiques, les agents actifs antidiarrhéiques, les antidiurétiques, les anti-estrogènes, les antimycotiques, les agents actifs antiglaucome, les antigonadotropines, les agents actifs contre la goutte, les substances séquestrant l'acide gallique, les dérivés de l'acide nicotinique, les hormones thyroïdiennes plasmatiques, les agents actifs antihypertoniques, les agents actifs contre le sur-fonctionnement de la thyroïde, les agents actifs contre le sous-fonctionnement de la thyroïde, les agents actifs contre l'hypotension, les agents actifs antipaludiques, les agents actifs contre la nausée, les agents actifs antinéoplasiques, les agents actifs antiparkinsoniens, les agents actifs pour le traitement de l'hypertrophie de la prostate, les agents actifs antiprotozoaires, les agents actifs antiprurigineux, les agents actifs antipsoriasiques, les agents actifs antipsychotiques, les antithrombotiques, les antitussifs, les agents actifs antiulcéreux, les uricostatiques, les agents actifs antiviraux, les bronchodilatateurs, les bloquants des canaux calciques, les cardiotoniques, les antagonistes de cholécystokinine, les agents actifs cholélitholytiques, les cholérétiques, les cholinergiques, les inhibiteurs de cholinestérase, les ré-activateurs de cholinestérase, les stimulants du SNC, les diurétiques, les agonistes des récepteurs de dopamine, les agents actifs contre les ectoparasites, les enzymes, les agents actifs inducteurs d'enzymes, les estrogènes stéroïdaux et non stéroïdaux, les inhibiteurs des sécrétions de l'estomac, les glucocorticoïdes, les agents actifs stimulant les gonades, les hormones gonadotropes, les inhibiteurs d'hormone de croissance, le facteur de libération d'hormone de croissance, les stimulants de croissance, les agents actifs hémolytiques, les antagonistes d'héparine, les agents hépatoprotecteurs, les agents pour le traitement de maladies hépatiques, les immunomodulateurs, les agents actifs immunosuppresseurs, les hormones stimulant la lactation, les agonistes de la LH-RH, les substances lipotropes, les agents contre le lupus érythémateux, les minéralocorticoïdes, les miotiques, les mucolytiques, les relaxants musculaires, les antagonistes de narcotiques, les agents actifs neuroprotecteurs, les nootropiques, les agents ophthalmiques, les hormones ovariennes, les oxytociques, les inhibiteurs de pepsine, les stimulants péristaltiques, les progestogènes, les inhibiteurs de prolactine, les prostaglandines, les inhibiteurs de protéase, les stimulants de la respiration, les sédatifs, les hypnotiques, les thrombolytiques, les uricosuriques, les vasodilatateurs, les agents vasoprotecteurs, les vitamines, les précurseurs de vitamines et les extraits de vitamines.

2. Préparation médicamenteuse sous la forme d'un film ou d'un cachet selon la revendication 1, **caractérisée en ce que** la préparation médicamenteuse est appropriée pour l'administration d'un ou de plusieurs agents actifs par le biais de la muqueuse de la bouche.

3. Préparation médicamenteuse sous la forme d'un film ou d'un cachet selon l'une quelconque des revendications précédentes, **caractérisée en ce que** la substance formant du dioxyde de carbone est contenue dans la préparation médicamenteuse en une quantité de 2 à 50 % en poids, de préférence de 5 à 30 % en poids et de manière particulièrement préférée de 7 à 20 % en poids, par rapport à la préparation médicamenteuse.

4. Préparation médicamenteuse sous la forme d'un film ou d'un cachet selon l'une quelconque des revendications précédentes, **caractérisée en ce qu'**elle contient au moins un amplificateur de perméation et/ou au moins une substance favorisant la circulation du sang.

5. Préparation médicamenteuse sous la forme d'un film ou d'un cachet selon la revendication 4, **caractérisée en ce que** l'amplificateur de perméation est choisi dans le groupe comprenant les acides gras saturés ou insaturés, les hydrocarbures, les alcools gras à chaîne linéaire ou ramifiée, le diméthylsulfoxyde, le propylène glycol, le décanol, le dodécanol, le 2-octyldodécanol, la glycérine, l'isopropylidène glycérol, le transcutol (= éther monoéthylique de diéthylène glycol), le DEET (= N,N-diéthyl-m-toluène-amide), le solkétal, l'éthanol ou d'autres alcools, le menthol et d'autres huiles éthérées ou des constituants d'huiles éthérées, le diéthanolamide de l'acide laurique, le D-alpha-tocophérol et le dexpanthénol.

6. Préparation médicamenteuse sous la forme d'un film ou d'un cachet selon la revendication 4, **caractérisée en ce que** la substance favorisant la circulation du sang est choisie dans le groupe comprenant le menthol, l'eucalyptol, l'extrait de ginkgo, l'huile de géranium, le camphre, l'huile de menthe crépue, l'huile de genévrier et le romarin.

7. Préparation médicamenteuse sous la forme d'un film ou d'un cachet selon l'une quelconque des revendications précédentes, **caractérisée en ce qu'**elle se décompose en 15 minutes, de préférence en 3 minutes et de manière particulièrement préférée en 60 secondes après l'introduction dans un milieu aqueux.

8. Préparation médicamenteuse sous la forme d'un film ou d'un cachet selon l'une quelconque des revendications précédentes, **caractérisée en ce que** le ou les polymères formant une matrice sont choisis dans le groupe comprenant l'alcool polyvinylique, les dérivés de cellulose, l'amidon et les dérivés d'amidon, la gélatine, la polyvinylpyrrolidone, la gomme arabique, le pullulane, les acrylates, le polyoxyde d'éthylène et les copolymères d'éther méthylvinylique et d'anhydride de l'acide maléique, le groupe des dérivés de cellulose comprenant de préférence l'hydroxypropylméthylcellulose, l'hydroxypropylcellulose, la carboxyméthylcellulose sodique, la méthylcellulose, l'hydroxyéthylcellulose et l'hydroxypropyléthylcellulose.

9. Préparation médicamenteuse sous la forme d'un film ou d'un cachet selon l'une quelconque des revendications 1 à 6, **caractérisée en ce que** le ou les polymères formant une matrice sont choisis dans le groupe comprenant les éthers de cellulose, de préférence l'éthylcellulose, ainsi que l'alcool polyvinylique, le polyuréthane, les polyméthacrylates, les polyméthacrylates de méthyle et les dérivés et copolymères des polymères susmentionnés.

10. Préparation médicamenteuse sous la forme d'un film ou d'un cachet selon l'une quelconque des revendications précédentes, **caractérisée en ce que** la préparation médicamenteuse contient un adjuvant qui confère à la préparation des propriétés mucoadhésives.

11. Préparation médicamenteuse sous la forme d'un film ou d'un cachet selon la revendication 10, **caractérisée en ce que** l'adjuvant est choisi dans le groupe comprenant l'acide polyacrylique, la carboxyméthylcellulose, l'hydroxyméthylcellulose, la méthylcellulose, la tragacanthe, l'acide alginique, la gélatine et la gomme arabique ou un mélange de ceux-ci.

12. Préparation médicamenteuse sous la forme d'un film ou d'un cachet selon la revendication 10, **caractérisée en ce que** la forme médicamenteuse est configurée à deux couches ou plus, seules la ou les couches orientées vers la muqueuse de la bouche ou se trouvant en contact avec celle-ci ayant des propriétés mucoadhésives.

13. Préparation médicamenteuse sous la forme d'un film ou d'un cachet selon la revendication 12, **caractérisée en ce que** les couches non mucoadhésives présentent une perméabilité plus faible pour l'agent actif ou les agents actifs.

14. Préparation médicamenteuse sous la forme d'un film ou d'un cachet selon l'une quelconque des revendications précédentes, **caractérisée en ce qu'**elle est de forme plate, la densité de cette préparation de forme plate étant de préférence comprise entre 0,3 g/cm³ et 1,7 g/cm³, de manière particulièrement préférée entre 0,5 g/cm³ et 1,5 g/cm³, et de manière préférée entre toutes entre 0,7 g/cm³ et 1,3 g/cm³.

15. Préparation médicamenteuse sous la forme d'un film ou d'un cachet selon l'une quelconque des revendications précédentes, **caractérisée en ce que** son épaisseur totale est de 5 µm à 10 mm, de préférence de 30 µm à 2 mm et de manière particulièrement préférée de 0,1 mm à 1 mm.

16. Préparation médicamenteuse sous la forme d'un film ou d'un cachet selon l'une quelconque des revendications précédentes, **caractérisée en ce qu'**elle présente une forme ronde ou elliptique ou ovale, ou une forme triangulaire, quadrangulaire ou multiangulaire, ou une forme arrondie irrégulière.

17. Préparation médicamenteuse sous la forme d'un film ou d'un cachet selon l'une quelconque des revendications précédentes, **caractérisée en ce qu'**elle se présente sous la forme d'une mousse solide, la densité de cette mousse solidifiée étant de préférence comprise entre 0,01 g/cm³ et 0,8 g/cm³, de manière particulièrement préférée entre 0,08 g/cm³ et 0,4 g/cm³, et de manière préférée entre toutes entre 0,1 g/cm³ et 0,3 g/cm³.

18. Préparation médicamenteuse sous la forme d'un film ou d'un cachet selon l'une quelconque des revendications précédentes, **caractérisée en ce que** la proportion de polymère de la matrice est d'au moins 3 % en poids et d'au plus 98 % en poids, de préférence de 7 à 80 % en poids, de manière particulièrement préférée de 20 à 50 % en poids, à chaque fois par rapport à la préparation totale.

19. Préparation médicamenteuse sous la forme d'un film ou d'un cachet selon l'une quelconque des revendications précédentes, **caractérisée en ce qu'**elle contient au moins un adjuvant, le ou les adjuvants étant choisis dans le groupe comprenant les charges, les colorants, les explosifs, les émulsifiants, les plastifiants, les édulcorants, les conservateurs, les stabilisateurs, les antioxydants et les aromatisants.

20. Préparation médicamenteuse sous la forme d'un film ou d'un cachet selon l'une quelconque des revendications précédentes, **caractérisée en ce qu'**elle contient au moins un aromatisant et/ou au moins un édulcorant et/ou au moins un plastifiant.

21. Préparation médicamenteuse sous la forme d'un film ou d'un cachet selon l'une quelconque des revendications précédentes, **caractérisée en ce que** l'agent actif est choisi dans le groupe comprenant l'acétaminophène, l'aspirine, le sulfosuccinate de phénolphthaléine-dioctylsodium, la phénylpropanolamine, la caféine, la théophylline, le chlorhydrate de diphénoxylate, la siméthicone, le tartrate d'ergotamine, l'halopéridol, le phénobarbitol, le méthyldopa, le méthylphénidate, la benzodiazépine, la phénothiazine, l'astermizole, le maléate de phéniramine, le maléate de chlorophéniramine, le maléate de bromophéniramine, le maléate de pyridamine, le succinate de doxylamine, le citrate de phényltoloxamine, le chlorhydrate de chlorocyclizine, le chlorhydrate de phénylpropanolamine, le bitartrate de phénylpropanolamine, le chlorhydrate de phényléphrine, le chlorhydrate de pseudoéphédrine, le sulfate de pseudoéphédrine, l'éphédrine, le propranolol, le disulfiram, le dextrométorphane, le chlorhydrate de dextrométorphane, la noscapine, le citrate de carbépentane, le chlorhydrate de chlophédianol, la tétracycline, la cléocine, la prednisone, la prednisolone, la colchicine, l'allopurinol, la phénytoïne sodium, le chlorure de cétylpyridinium, le naproxène, le lopéramide, la famotidine, le phosphate de codéine, le sulfate de codéine, la morphine, le chlorure de potassium, le chlorure de zinc, la cholestyramine, le N-acétylprocaïnamide, la guaïfénésine, la sélégéline, l'atipamézole, la nicotine, la quinine, le bruzine, le dextrométhorphane, la caféine, la théophylline, la théobromine, le métamizole, le sulfate de magnésium, la zopliclone, le zolpidem, les agents antibactériens à base d'acide pyridonecarboxylique, l'énoxacine, l'acide pipémidique, la ciproflaxine, l'ofloxacine, la péfloxacine, le zonisamide, l'érythromycine, la pénicilline, la céphalosporine, la chloropromazine, la sulpyrine et la cimétidine.

22. Utilisation d'une substance formant du dioxyde de carbone, ou d'une combinaison de telles substances, en tant qu'additif masquant le goût dans une préparation médicamenteuse sous la forme d'un film ou d'un cachet, appropriée pour l'administration orale, qui comprend une matrice qui est formée par au moins un polymère formant une matrice et dans laquelle au moins un agent actif à goût amer est dissous ou dispersé, qui est résorbable par la muqueuse de la bouche ou dans l'estomac et/ou dans l'intestin, la substance formant du dioxyde de carbone étant choisie dans le groupe constitué par l'hydrogénocarbonate de sodium, le carbonate de sodium, le carbonate de potassium et l'hydrogénocarbonate de potassium, et ne se présentant pas en combinaison avec un composant acide, et l'agent actif étant choisi dans le groupe comprenant les antipyrétiques, les laxatifs, les anorexigènes, les antacides, les antiasthmatiques, les antidiurétiques, les agents contre les flatulences, les agents contre la migraine, les agents psychopharmaceutiques, les spasmolytiques, les sédatifs, les antihyperkinétiques, les tranquillisants, les antihistaminiques, les agents décongestionnants, les bloquants des récepteurs bêta, les agents de désaccoutumance de l'alcool, les compléments de fluor, les antibiotiques locaux, les compléments corticostéroïdes, les agents contre la formation de goitres, les antiépileptiques, les agents contre la déshydratation, les antiseptiques, l'acétaminophène, le naproxène, les agents actifs gastro-intestinaux, les alcaloïdes, les compléments d'oligoéléments, les réducteurs de cholestérol et de lipides, les agents antiarrhythmiques, les expectorants, les xanthines, les pyrazolones, les agonistes α-adrénergiques, les agonistes β-adrénergiques, les bloquants α-adrénergiques, les inhibiteurs d'aldose-réductase, les anaboliques, les analgésiques narcotiques, les androgènes, les anthelminthiques, les agents actifs anti-acné, les antiallergiques, les agents actifs amibicides, les anti-androgènes, les agents actifs contre l'angine de poitrine, les agents actifs anti-artériosclérotiques, les antibiotiques, les anticholinergiques, les anticonvulsifs, les antidépresseurs, les agents antidiabétiques, les agents actifs antidiarrhéiques, les antidiurétiques, les anti-estrogènes, les antimycotiques, les agents actifs antiglaucome, les antigonadotropines, les agents actifs contre la goutte, les substances séquestrant l'acide gallique, les dérivés de l'acide nicotinique, les hormones thyroïdiennes plasmatiques, les agents actifs antihypertoniques, les agents actifs contre le sur-fonctionnement de la thyroïde, les agents actifs contre le sous-fonctionnement de la thyroïde, les agents actifs contre l'hypotension, les agents actifs antipaludiques, les agents actifs contre la nausée, les agents actifs antinéoplasiques, les agents actifs antiparkinsoniens, les agents actifs pour le traitement de l'hypertrophie de la prostate, les agents actifs antiprotozoaires, les agents actifs antiprurigineux, les agents actifs antipsoriasiques, les agents actifs antipsychotiques, les antithrombotiques, les antitussifs, les agents actifs antiulcéreux, les uricostatiques, les agents actifs antiviraux, les bronchodilatateurs, les bloquants des canaux calciques, les cardiotoniques, les antagonistes de cholécystokinine, les agents actifs cholélitholytiques, les cholérétiques, les cholinergiques, les inhibiteurs de cholinestérase, les ré-activateurs de cholinestérase, les stimulants du SNC, les diurétiques, les agonistes des récepteurs de dopamine, les agents actifs contre les ectoparasites, les enzymes, les agents actifs inducteurs d'enzymes, les estrogènes stéroïdaux et non stéroïdaux, les inhibiteurs des sécrétions de l'estomac, les glucocorticoïdes, les agents actifs stimulant les gonades, les hormones gonadotropes, les inhibiteurs d'hormone de croissance, le facteur de libération d'hormone de croissance, les stimulants de croissance, les agents actifs hémolytiques, les antagonistes d'héparine, les agents hépatoprotecteurs, les agents pour le traitement de maladies hépatiques, les immunomodulateurs, les agents actifs immunosuppresseurs, les hormones stimulant la lactation, les agonistes de la LH-RH, les substances lipotropes, les agents contre le lupus érythémateux, les minéralocorticoïdes, les miotiques, les mucolytiques, les relaxants musculaires, les antagonistes de narcotiques, les agents actifs neuroprotecteurs, les nootropiques, les agents ophthalmiques, les hormones ovariennes, les oxytociques, les inhibiteurs de pepsine, les stimulants péristaltiques, les progestogènes, les inhibiteurs de prolactine, les prostaglandines, les inhibiteurs de protéase, les stimulants de la respiration, les sédatifs, les hypnotiques, les thrombolytiques, les uricosuriques, les vasodilatateurs, les agents vasoprotecteurs, les vitamines, les précurseurs de vitamines et les extraits de vitamines.
